# EUROPÄISCHE PATENTSCHRIFT

(11) **EP 1 605 843 B1**
(45) Veröffentlichungstag und Bekanntmachung des Hinweises auf die Patenterteilung: **09.05.2007**
(21) Anmeldenummer: 03816328.3
(22) Anmeldetag: 21.03.2003
(51) Int. Cl.: A61B 17/58, B23D 29/02

(54) **SCHNEIDE- UND UMFORMVORRICHTUNG**
CUTTING AND FORMING DEVICE
DISPOSITIF DE COUPE ET DE FORMAGE

(43) Veröffentlichungstag der Anmeldung: 21.12.2005
(73) Patentinhaber: Synthes GmbH, 4436 Oberdorf (CH)
(72) Erfinder: MATTHYS-MARK, Romano, CH-7272 Davos Clavadel (CH); GELPKE, Hans, CH-8542 Wiesendangen (CH)
(74) Vertreter: Lusuardi, Werther
(86) Internationale Anmeldenummer: PCT/CH2003/000183
(87) Internationale Veröffentlichungsnummer: WO 2004/082492

(56) Entgegenhaltungen:
- EP-A- 0 928 602
- DE-B- 1 277 515
- DE-U- 8 708 571
- US-A- 3 315 669
- US-A- 5 980 547

## Beschreibung

Die Erfindung bezieht sich auf eine Vorrichtung zum Schneiden eines Drahtes und gleichzeitigem Umformen einer Drahtendschutzhülse, gemäss dem Oberbegriff des Patentanspruchs 1. Eine derartige Vorrichtung ist aus dem US-A-3 315 669 bekannt.

Beispielsweise bei der Ausführung eines chirurgischen Eingriffes oder zur Stabilisierung von Knochen oder Knochenfragmenten mittels eines Fixateur externe werden häufig Kirschnerdrähte oder ähnliche Fixationselemente eingesetzt. Diese müssen je nach Anwendung nach ihrer Fixierung am Knochen oder Knochenfragment auf eine gewünschte Länge gebracht werden. Zur Kürzung der Kirschnerdrähte oder Fixationselemente werden diese mit Drahtschneidewerkzeugen durchgetrennt.

Aus der US 4,051,596 HOFMANN ist ein Drahtschneidewerkzeug mit zwei konzentrisch ineinander rotierbaren Hülsen bekannt. Diese bekannte Vorrichtung umfasst Schneidkanten, welche zur Längsachse der Hülsen konzentrisch sind. Nachteilig an dieser bekannten Drahtschneidevorrichtung ist, dass bei deren Verwendung während eines chirurgischen Eingriffes der am Knochen oder Knochenfragment verbleibende Teil des Kirschnerdrahtes nach dem Durchtrennen an der Schnittstelle gequetscht ist und einen Grat aufweist oder scharfe Kanten respektive Spitzen entstehen, so dass an den angrenzenden Weichteilen durch den Kirschnerdraht Irritationen hervorgerufen werden können.

Hier will die Erfindung Abhilfe schaffen. Der Erfindung liegt die Aufgabe zugrunde, eine Vorrichtung zu schaffen, welche mittels mindestens einer kreisförmig um den Kirschnerdraht herum bewegbaren Schnittkante einen gratfreien Schnitt durch den zu trennenden Kirschnerdraht oder ein anderes zu trennendes Fixationselement ermöglicht und gleichzeitig das Aufpressen einer Drahtendschutzhülse auf das Ende des getrennten Kirschnerdrahtes gestattet.

Die Erfindung löst die gestellte Aufgabe mit einer Vorrichtung zum Schneiden eines Drahtes und gleichzeitigem Umformen einer Drahtendschutzhülse, welche die Merkmale des Anspruchs 1 aufweist.

Die durch die Erfindung erreichten Vorteile sind im wesentlichen darin zu sehen, dass dank der erfindungsgemässen Schneidvorrichtung
a) eine gratfreie Trennung eines Kirschnerdrahtes oder eines anderen Fixationselementes ermöglicht wird;
b) beim Trennvorgang keine scharfen Kanten oder Spitzen entstehen; und
c) durch die Führung des Kirschnerdrahtes in einer Bohrung eine zur Längsachse senkrecht stehende Trennfläche herstellbar ist und das hintere Ende des Kirschnerdrahtes ohne Nachbearbeitung, wie beispielsweise das Entfernen von scharfen Kanten oder Spitzen mit einer Drahtendschutzhülse versehen werden kann.

Zwei Hülsen sind derart ausgestaltet, dass die Bohrung in der inneren Hülse zur Aufnahme eines Führungs- oder Kirschnerdrahtes dient und einen Durchmesser d aufweist und die Verengung der Bohrung in der äusseren Hülse zur Aufnahme einer Drahtendschutzhülse dient und einen Durchmesser D aufweist, wobei D > d ist. Durch diese Ausgestaltung der Bohrung in der inneren Hülse ist erreichbar, dass am vorderen Ende der inneren Hülse eine den Führungsdraht umfassende kreisförmige Schneidkante gebildet wird, während durch die Ausgestaltung der Verengung die Drahtendschutzhülse in der äusseren Hülse radial fest gehalten wird, so dass durch die Wandung der Zentralbohrung der Drahtendschutzhülse am inneren Ende der Drahtendschutzhülse eine zweite, den Führungsdraht umfassende kreisförmige Schneidkante gebildet wird.

In einer Ausführungsform sind die Abstände a und b so ausgestaltet, dass a ≥ b ist. Durch die verschiedenen Exzentrizitäten der Bohrung in der inneren Hülse und der Verengung lässt sich bei einem Rotieren der inneren Hülse relativ zur äusseren Hülse ein Führungsdraht trennen und gleichzeitig die Drahtendschutzhülse auf das Ende des Führungsdrahtes aufquetschen.

In wiederum einer weiteren Ausührungsform ist mindestens eine der Hülsen an ihrem hinteren Ende mit einem quer zu den Bohrungsachsen angeordneten Betätigungshebel ausgestattet ist, wodurch grössere Scherkräfte auf den Führungsdraht ausübbar sind.

In einer anderen Ausführungsform der erfindungsgemässen Vorrichtung umfasst diese zusätzlich eine Drahtendschutzhülse mit einem hinteren Ende, einem vorderen Ende und einer Zentralbohrung. Die Drahtendschutzhülse ist parallel zur Längsachse in die Verengung einschiebbar und das hintere Ende der Drahtendschutzhülse axial an das vordere Ende der inneren Hülse zur Anlage bringbar. Ferner ist die Zentralbohrung der Drahtendschutzhülse am hinteren Ende mit einer zur Zentralbohrung konzentrischen, zweiten Schnittkante versehen. Da die Längsachse der Verengung und die Bohrungsachse der Bohrung in der inneren Hülse exzentrisch zur Rotationsachse der inneren Hülse, welche durch die mit der Bohrungsachse der Bohrung in der äusseren Hülse zusammenfallende Längsachse der kreiszylindrischen inneren Hülse gebildet wird, angeordnet sind, erfolgt bei Verdrehen der inneren Hülse relativ zur äusseren Hülse eine Abscherung des Kirschnerdrahtes zwischen der ersten und der zweiten Schnittkante.

Damit ist der weitere Vorteil erreichbar, dass falls die Drahtendschutzhülse nicht verwendet wird, der Führungsdraht nicht getrennt werden kann. Somit ist die zusätzliche Sicherheit erreichbar, dass der Führungsdraht nur gekürzt werden kann, wenn die Drahtendschutzhülse in die Vorrichtung eingefügt ist.

Die erfindungsgemässe Vorrichtung umfasst ein Gehäuse mit einer Zentralachse und einem das Gehäuse koaxial zur Zentralachse durchdringenden Hohlraum, worin die beiden Hülsen aufnehmbar sind. Ferner umfasst der Hohlraum am vorderen Ende des Gehäuses eine Bohrung mit einer zur Zentralachse des Gehäuses parallelen, einen Abstand c zu dieser aufweisenden Längsachse. Der Durchmesser dieser Bohrung entspricht dem Aussendurchmesser der Drahtendschutzhülse, so dass zwischen der Drahtendschutzhülse und dem Kirschnerdraht mittels Rotieren der äusseren Hülse im Hohlraum des Gehäuse eine Pressverbindung herstellbar ist.

In wiederum einer weiteren Ausführungsform der erfindungsgemässen Vorrichtung umfasst eine der Hülsen einen Mitnehmer und die andere Hülse eine Aussparung, wobei der Mitnehmer bei einer Rotation der Hülsen relativ zueinander um einen Drehwinkel α innerhalb der Aussparung frei bewegbar ist. Der Drehwinkel α liegt vorzugsweise in einem Bereich zwischen 90° und 180°. Damit ist erreichbar, dass nach Beenden des Schnittvorganges Mitnehmer und Anschlagfläche in der Aussparung aneinander anliegen, so dass die beiden Hülsen nicht weiter relativ zueinander rotierbar sind, aber zusammen gegenüber dem Gehäuse rotierbar sind. Durch die Rotation der beiden Hülsen relativ zum Gehäuse ist dann der Pressvorgang zwischen Drahtendschutzhülse und Kirschnerdraht ausführbar.

Die Exzentrizitäten a, b und c liegen vorzugsweise in den folgenden Bereichen:
- Exzentrizität a zwischen 0,2 mm und 0,8 mm;
- Exzentrizität b zwischen 0,2 mm und 0,8 mm; und
- Exzentrizität c zwischen 0,05 mm und 0,4 mm.

In einer anderen Ausführungsform der erfindungsgemässen Schneidvorrichtung wird durch die Verengung in der Bohrung in der äusseren Hülse ein Absatz mit einer zur Bohrungsachse senkrecht stehenden, ebenen Auflagefläche gebildet, so dass das vordere Ende der inneren Hülse an dieser Auflagefläche zur Anlage bringbar ist. Durch diese Ausgestaltung ist erreichbar, dass am Austritt der Bohrung in der inneren Hülse eine erste kreisförmige Schnittkante herstellbar ist und am Übergang der Auflagefläche zur Verengung in der äusseren Hülse eine zweite kreisförmige, zur Längsachse der Verengung konzentrische Schnittkante herstellbar ist. Durch Verdrehen der beiden Hülsen relativ zueinander ist wegen der Exzentrizität der Bohrungsachse der Bohrung in der inneren Hülse und der Längsachse der Verengung ein Abscheren des Kirschnerdrahtes ausführbar.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 einen Längsschnitt durch eine Ausführungsform der erfindungsgemässen Vorrichtung;
Fig. 2 den Schnitt B-B durch die in Fig. 1 dargestellte Ausführungsform der erfindungsgemässen Vorrichtung;
Fig. 3 eine Ansicht von A der in Fig. 1 dargestellten Ausführungsform der erfindungsgemässen Vorrichtung mit zum Einführen von Drahtendschutzhülse und Kirschnerdraht positionierten Hülsen und Gehäuse;
Fig. 4 eine Ansicht von A der in Fig. 1 dargestellten Ausführungsform der erfindungsgemässen Vorrichtung mit gegeneinander verdrehten Hülsen; und
Fig. 5 eine Ansicht von A der in Fig. 1 dargestellten Ausführungsform der erfindungsgemässen Vorrichtung nach Verdrehen der Hülsen relativ zueinander und gegenüber dem Gehäuse.

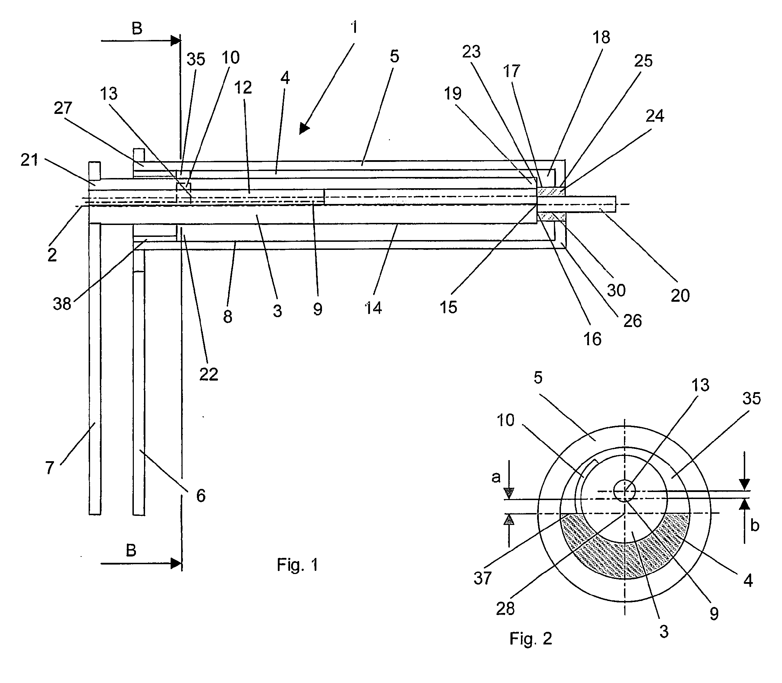

In Fig. 1 ist eine Ausführungsform der erfindungsgemässen Vorrichtung 1 dargestellt, welche ein hohlzylindrisches Gehäuse 5 mit einem eine Zentralachse 2 aufweisenden Hohlraum 8 sowie zwei Hülsen 3;4 umfasst, welche im Hohlraum 8 derart angeordnet sind, dass sie um die Zentralachse 2 rotierbar sind. Die äussere, eine erste Längsachse 28 aufweisende Hülse 4 ist mit einer exzentrischen, eine erste Bohrungsachse 9 aufweisenden, die Hülse 4 longitudinal durchdringenden ersten Bohrung 14 ausgestaltet. In dieser ersten Bohrung 14 ist die innere Hülse 3 koaxial um die erste Bohrungsachse 9 rotierbar gelagert. Aussen ist die äussere Hülse 4 konzentrisch zur ersten Längsachse 28 kreiszylindrisch ausgestaltet. Die innere Hülse 3 ist aussen zur ersten Bohrungsachse 9 koaxial kreiszylindrisch ausgestaltet und weist eine zweite Bohrung 12 mit einer zur ersten Bohrungsachse 9 exzentrischen zweiten Bohrungsachse 13 auf. Diese zweite Bohrung 12 dient zur Aufnahme eines Kirschnerdrahtes 20 oder andern Fixationselementes. Die erste Bohrung 14 in der äussere Hülse 4 weist am vorderen Ende 18 der äusseren Hülse 4 eine Verengung 17 auf, wodurch in der ersten Bohrung 14 eine senkrecht zur ersten Bohrungsachse 9 den ersten Bohrung 14 stehende Auflagefläche 23 gebildet wird, woran das vordere Ende 19 der inneren Hülse 3 zur Anlage bringbar ist.

Die erste Schnittkante15 wird durch den kreislinienförmigen, scharfkantigen Rand am Austritt der zweiten Bohrung 12 am vorderen Ende 19 der inneren Hülse 3 gebildet. In der hier dargestellten Ausführungsform der erfindungsgemässen Vorrichtung wird eine Drahtendschutzhülse 24 in die Verengung 17 in der äusseren Hülse 4 eingeführt. Die Drahtendschutzhülse 24 ist mit einer Zentralbohrung 30 zur Aufnahme eines Krischnerdrahtes 20 ausgestaltet, wobei die Enden der Zentralbohrung 30 scharfkantig ausgebildet sind. Dadurch wird am Ende der Drahtendschutzhülse 24 eine zweite, gegen die erste Schnittkante 15 gerichtete Schnittkante 16 gebildet.

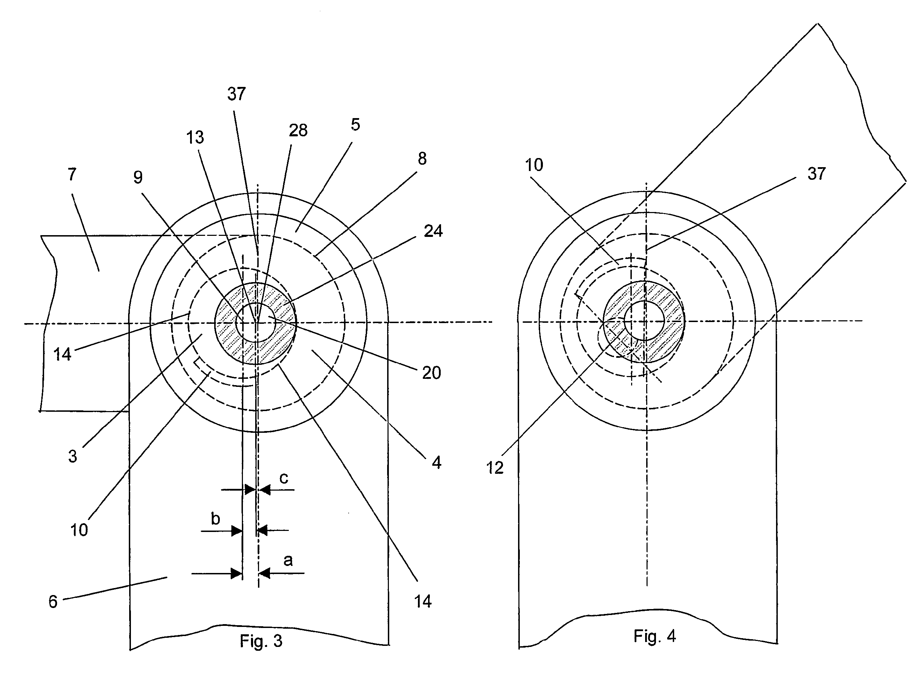

Durch die Exzentrizität a der ersten Bohrungsachse 9 der ersten Bohrung 14, welche zur Aufnahme der inneren Hülse 3 dient, gegenüber der zweiten Längsachse 11, sowie die Exzentrizität b der zweiten Bohrungsachse 13 der zweiten Bohrung 12, welche zur Aufnahme des Kirschnerdrahtes 20 dient, und der ersten Bohrungsachse 9 lässt sich die innere Hülse 3 relativ zur äusseren Hülse 4 in eine rotative Position bringen, in welcher die zweite Bohrung 12 mit der Zentralbohrung 30 in der Drahtendschutzhülse 24 fluchtet. In dieser Position der inneren Hülse 3 (Fig. 3) kann der Kirschnerdraht 20 durch die Zentralbohrung 30 in der Drahtendschutzhülse 24 in die zweite Bohrung 12 in der inneren Hülse 3 eingeführt werden.

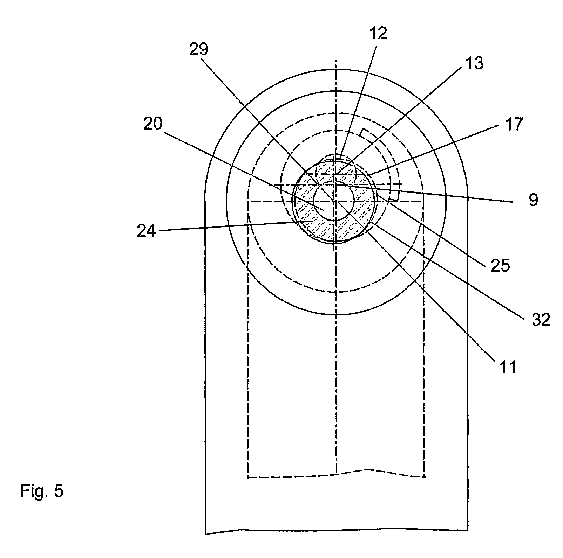

Durch Verdrehen der beiden Hülsen 3;4 relativ zueinander (Fig. 4 und 5) wird der Kirschnerdraht 20 mittels der durch die zueinander exzentrisch rotierenden Schnittkanten 15;16 verursachten Scherbewegung zwischen den Schnittkanten 15;16 getrennt.

Zur Bedienung der beiden Schneidelemente 3;4 sind am hinteren Ende 27 des Gehäuses 5 ein senkrecht zur Hohlraumlängsachse 9 angeordneter Haltegriff 6 und am hinteren Ende 21 der inneren Hülse 3 je ein ebenfalls senkrecht zur Hohlraumlängsachse 9 angeordneter Hebel 7 angebracht.

Neben dem Schneiden der Kirschnerdrahtes 20 ist mit der in den Fig. 1 bis 5 dargestellten Ausführungsform der erfindungsgemässen Vorrichtung 1 im selben Arbeitsgang eine Drahtendschutzhülse 24 am hinteren Ende des Kirschnerdrahtes 20 befestigbar. Die Befestigung erfolgt durch Anpressen der Seitenwand 32 der Drahtendschutzhülse 24 an den Kirschnerdraht 20 mittels der exzentrischen Anordnung der Verengung 17 an der äusseren Hülse 4 und der dritten Bohrung 25 am vorderen Ende 26 des Gehäuses 5. Durch die Ausgestaltung der Exzentrizitäten a, b und c mit der Bedingung a - b = c (Fig. 3) ist erreichbar, dass in der in Fig. 3 dargestellten relativen Position von innerer Hülse 3, äusserer Hülse 4 und Gehäuse 5 sowohl die Drahtendschutzhülse 24 in die dritte Bohrung 25 am vorderen Ende 26 des Gehäuses 5 und in die Verengung 17 am vorderen Ende 19 der äusseren Hülse 4 koaxial zur zweiten Bohrungsachse 13 der zweiten Bohrung 12 als auch der Kirschnerdraht 20 durch die Zentralbohrung 30 der Drahtendschutzhülse 24 koaxial zur zweiten Bohrungsachse 13 in die zweite Bohrung 12 einführbar sind. Die in Fig. 3 dargestellte Position zeichnet sich dadurch aus, dass die innere Hülse, die äussere Hülse 4 so positioniert sind, dass die drei Exzentrizitäten a, b und c auf einer Geraden liegen und die Bedingung a = b + c erfüllen (Fig. 3).

Die äussere Hülse 4 ist an ihrem hinteren Ende 22 als Halbschale ausgeführt, so dass am hinteren Ende 22 der äusseren Hülse 4 eine Aussparung 35 mit zwei zur Zentralachse 2 parallelen Anschlagflächen 37 gebildet wird. Ein am hinteren Ende 22 der inneren Hülse 3 radial vorstehender Mitnehmer 10 kommt bei einem Drehwinkel zwischen der inneren und der äusseren Hülse 3;4 von 135° an einer dieser Anschlagflächen 37 zur Anlage, so dass bei einem weiteren Drehen des Betätigungshebels 7 relativ zum Haltegriff 6 die beiden Hülsen 3;4 zusammen relativ zum Gehäuse 5 gedreht werden (Fig. 5), wodurch die Verengung 17 mit der darin eingeführten Drahtendschutzhülse 24 gegen über der dritten Bohrung 25 im Gehäuse 5 verdreht wird. Durch die exzentrische Anordnung der dritten Längsachse 29 der dritten Bohrung 25 relativ zur zweiten Längsachse 11 der Verengung 17 wird die Drahtendschutzhülse 24 durch die Wände der dritten Bohrung 25 und der Verengung 17 gequetscht und auf dem in der Zentralbohrung 30 der Drahtendschutzhülse 24 eingeführten Kirschnerdraht 20 fixiert.

## Patentansprüche

1. Vorrichtung (1) zum Schneiden eines Drahtes und gleichzeitigem Umformen einer Drahtendschutzhülse (24) mit
A) einer äusseren, eine erste Längsachse (28) aufweisenden Hülse (4), welche ein vorderes Ende (18), ein hinteres Ende (22) und eine die Hülse (4) longitudinal durchdringende, eine erste Bohrungsachse (9) aufweisenden ersten Bohrung (14) umfasst; und
B) einer inneren, in der ersten Bohrung (14) koaxial zur ersten Bohrungsachse (9) rotierbaren Hülse (3), welche ein vorderes Ende (19), ein hinteres Ende (21) und eine die Innere Hülse (3) longitudinal durchdringende, eine zweite Bohrungsachse (13) aufweisende zweite Bohrung (12) umfasst, wobei
C) die erste Bohrung (14) am vorderen Ende (18) der äusseren Hülse (4) eine Verengung (17) mit einer zweiten Längsachse (11) umfasst, wobei die zweite Längsachse (11) zur ersten Bohrungsachse (9) parallel ist und eine erste Exzentrizität a > 0 zu dieser aufweist; und
D) die zweite Bohrungsachse (13) der zweiten Bohrung (12) zur ersten Bohrungsachse (9) der ersten Bohrung (14) parallel verläuft und eine zweite Exzentrizität b > 0 zu dieser aufweist, wobei
E) die zweite Bohrung (12) in der inneren Hülse (3) zur Aufnahme eines Führungs- oder Kirschnerdrahtes (20) dient und einen ersten Durchmesser d aufweist;
F) die Verengung (17) zur Aufnahme einer Drahtendschutzhülse (24) dient und einen zweiten Durchmesser D aufweist;
G) D > d ist;
H) die Wandung der zweiten Bohrung (12) am vorderen Ende (19) der inneren Hülse (3) eine kreisförmige, zur zweiten Bohrungsachse (13) konzentrische Schnittkante (15) umfasst; **dadurch gekennzeichnet, dass**
I) die Vorrichtung (1) ein Gehäuse (5) mit einer Zentralachse (2), einem vorderen Ende (26), einem hinteren Ende (27) und einem das Gehäuse (5) parallel zur Zentralachse (2) durchdringenden Hohlraum (8) zur Aufnahme der äusseren Hülse (4) umfasst; und
K) der Hohlraum (8) am vorderen Ende (26) des Gehäuses (5) eine dritte Bohrung (25) mit einer zur Zentralachse (2) parallelen, eine dritte Exzentrizität c > 0 zu dieser aufweisenden dritten Längsachse (29) umfasst.

2. Vorrichtung nach Anspruch 1, **dadurch gekennzeichnet, dass** a > b ist.

3. Vorrichtung nach Anspruch 1 oder 2, **dadurch gekennzeichnet, dass** mindestens eine der Hülsen (3;4) an ihrem hinteren Ende (21;22) mit einem quer zu den Bohrungsachsen (9;13) angeordneten Betätigungshebel (7) ausgestattet ist.

4. Vorrichtung nach einem der Ansprüche 1 bis 3, **dadurch gekennzeichnet, dass** sie zusätzlich eine Drahtendschutzhülse (24) mit einem hinteren Ende (33), einem vorderen Ende (34) und einer Zentralbohrung (30) umfasst.

5. Vorrichtung nach einem der Ansprüche 1 bis 4, **dadurch gekennzeichnet, dass** durch die Verengung (17) ein Absatz (31) mit einer zur ersten Bohrungsachse (9) senkrecht stehenden, ebenen Auflagefläche (23) gebildet wird und das vordere Ende (19) der inneren Hülse (3) an dieser Auflagefläche (23) anliegt.

6. Vorrichtung nach Anspruch 4 oder 5, **dadurch gekennzeichnet, dass** die Drahtendschutzhülse (24) parallel zur Längsachse (11) in die Verengung (17) einschiebbar ist und das hintere Ende (33) der Drahtendschutzhülse (24) axial an das vordere Ende (19) der inneren Hülse (3) zur Anlage bringbar ist.

7. Vorrichtung nach einem der Ansprüche 4 bis 6, **dadurch gekennzeichnet, dass** die Wandung der Zentralbohrung (30) in der Drahtendschutzhülse (24) am hinteren Ende (33) eine zur Zentralbohrung (30) konzentrische, zweite Schnittkante (16) umfasst.

8. Vorrichtung nach einem der Ansprüche 1 bis 7, **dadurch gekennzeichnet, dass** der Durchmesser der dritten Bohrung (25) dem Aussendurchmesser der Drahtendschutzhülse (24) entspricht, so dass zwischen der Drahtendschutzhülse (24) und dem Draht (20) mittels Rotieren der äusseren Hülse (4) im Hohlraum (8) eine Pressverbindung herstellbar ist.

9. Vorrichtung nach einem der Ansprüche 1 bis 8, **dadurch gekennzeichnet, dass** der Betätigungshebel (7) am hinteren Ende (21) der inneren Hülse (3) befestigbar ist.

10. Vorrichtung nach Anspruch 9, **dadurch gekennzeichnet, dass** eine der Hülsen (3;4) einen Mitnehmer (10) und die andere Hülse (3;4) eine Aussparung (35) umfasst, wobei der Mitnehmer (10) bei einer Rotation der Hülsen (3;4) relativ zueinander um einen Drehwinkel α innerhalb der Aussparung (35) frei bewegbar ist.

11. Schneidvorrichtung nach Anspruch 10, **dadurch gekennzeichnet, dass** der Drehwinkel α in einem Bereich zwischen 90° und 180° liegt.

12. Vorrichtung nach einem der Ansprüche 1 bis 11, **dadurch gekennzeichnet, dass** die zweite Bohrungsachse (13) zur zweiten Längsachse (11) eine Exzentrizität X aufweist, welcher zwischen 0,2 mm und 0,8 mm beträgt.

13. Vorrichtung nach Anspruch 12, **dadurch gekennzeichnet, dass** die Exzentrizität X zwischen 0,4 mm und 0,6 mm beträgt.

14. Schneidvorrichtung nach einem der Ansprüche 1 bis 13, **dadurch gekennzeichnet, dass** die dritte Exzentrizität c zwischen 0,05 mm und 0,5 mm beträgt.

15. Schneidvorrichtung nach Anspruch 14, **dadurch gekennzeichnet, dass** der Abstand c zwischen 0,1 mm und 0,3 mm beträgt.

## Claims

1. Device (1) for cutting a wire and, at the same time, forming a sleeve for protecting the end of the wire with
A) an outer sleeve (4), which has a first longitudinal axis (28) and comprises a front end (18), a rear end (22) and a first borehole (14) having a first borehole axis (9) and passing longitudinally through the sleeve (4) and
B) an inner sleeve (3), which can be rotated in the first borehole (14) coaxially to the first borehole axis (9) and comprises a front end (19), a rear end (21) and a second borehole (12) having a second borehole axis (13) passing through the inner sleeve (3) longitudinally,
C) the first borehole (14), at the front end (18) of the outer sleeve (4), has a constriction (17) with a second longitudinal axis (11), whereby the second longitudinal axis (11) is parallel to the first borehole axis (9) and is at a first eccentricity a > 0 from the latter; and
D) the second borehole axis (13) of the second borehole (12) extends parallel to the first borehole axis (9) of the first borehole (14) at a second eccentricity b > 0 therefrom, whereby
E) the second borehole (12) in the inner sleeve (3) is suitable for accommodating a guiding or Kirschner wire (20) and has a first diameter d;
F) the constriction (17) is suitable for accommodating a wire end protecting sleeve (24) for and has a second diameter D,
G) D > d;
H) the wall of the second borehole (12), at the front end (19) of the inner sleeve (3) has a circular cutting edge (15), which is concentric with the second borehole axis (13),
**characterized in that**
I) the device (1) comprises a housing (5) with a central axis (2), a front end (26), a rear end (27) and a cavity (8), passing through the housing (5) parallel to the central axis (2), for accommodating the outer sleeve (4) and
K) the cavity (8) at the front end (26) of the housing (5) includes a third borehole (25) with a third longitudinal axis (29), extending parallel to the central axis (2) at a third eccentricity c > 0 from the latter.

2. The device of claim 1, **characterized in that** a > b.

3. The device of one of the claims 1 or 2, **characterized in that** at least one of the sleeves (3; 4) is equipped at its rear end (21; 22) with an actuating lever (7), which is disposed transversely to the borehole axes (9; 13).

4. The device of one of the claims 1 to 3, **characterized in that** it additionally comprises a wire end protection sleeve (24), with a rear end (33), a front end (34) and a central borehole (30).

5. The device of one of the claims 1 to 4, **characterized in that** a shoulder (31) with a flat supporting surface (23), perpendicular to the first borehole axis (9), is formed by the constriction (17) and the front end (19) of the inner sleeve (3) rests on this supporting surface (23).

6. The device of claims 4 or 5, **characterized in that** the wire end protection sleeve (24) can be pushed parallel to the longitudinal axis (11) into the constriction (17) and the rear end (33) of the wire end protection sleeve (24) can be brought axially into contact with the front end (19) of the inner sleeve (3).

7. The device of one of the claims 4 to 6, **characterized in that** the walls of the central borehole (30) in the wire end protection sleeve (24) has at the rear end (33) a central borehole (30), which embraces the second cutting edge (16) concentrically.

8. The device of one of the claims 1 to 7, **characterized in that** the diameter of the third borehole (25) corresponds to the external diameter of the wire end protection sleeve (24), so that a compression connection may be produced between the wire end protection sleeve (29) and the wire (20) by rotating the outer sleeve (4) in the cavity (8).

9. The device of one of the claims 1 to 8, **characterized in that** the actuating lever (7) may be fastened at the rear end (21) of the inner sleeve (3).

10. The device of claim 9, **characterized in that** one of the sleeves (3; 4) surrounds a catch (10) and the other sleeve (3; 4) a recess (35), the catch (10) being freely movable about an angle of rotation α within the recess (35) when the sleeves (3; 4) are rotated relative to one another.

11. The cutting device of claim 10, **characterized in that** the angle of rotation α ranges from 90° to 180°.

12. The device of one of the claims 1 to 11, **characterized in that** the second borehole axis (13) is at an eccentricity X, which is between 0,2 mm and 0,8 mm, from the longitudinal axis (11).

13. The device of claim 12, **characterized in that** the eccentricity X is between 0,4 mm and 0,6 mm.

14. Cutting device of one of the claims 1 to 13, **characterized in that** the third eccentricity c is between 0,05 mm and 0,5 mm.

15. The cutting device of claim 14, **characterized in that** the third eccentricity c is between 0,1 mm and 0,3 mm.

## Revendications

1. Dispositif (1) pour couper un fil métallique et déformer en même temps une gaine protectrice d'extrémité de fil (24), comprenant
A) une gaine externe (4) présentant un premier axe longitudinal (28), laquelle comprend une extrémité avant (18), une extrémité arrière (22) et un premier alésage (14), présentant un premier axe d'alésage (9), traversant la gaine (4) dans le sens longitudinal ; et
B) une gaine interne (3) pouvant tourner dans le premier alésage (14) coaxialement au premier axe d'alésage (9), laquelle comprend une extrémité avant (19), une extrémité arrière (21) et un deuxième alésage (12), présentant un deuxième axe d'alésage (13), traversant la gaine interne (3) dans le sens longitudinal, dans lequel
C) le premier alésage (14) comprend, au niveau de l'extrémité avant (18) de la gaine externe (4), un rétrécissement (17) présentant un deuxième axe longitudinal (11), le deuxième axe longitudinal (11) étant parallèle au premier axe d'alésage (9) et présentant une première excentricité a > 0 par rapport à celui-ci ; et
D) le deuxième axe d'alésage (13) du deuxième alésage (12) s'étendant parallèlement au premier axe d'alésage (9) du premier alésage (14) et présentant une deuxième excentricité b > 0 par rapport à celui-ci, dans lequel
E) le deuxième alésage (12) dans la gaine interne (3) sert à loger un fil-guide ou une broche de Kirschner (20) et présente un premier diamètre d ;
F) le rétrécissement (17) sert à loger une gaine protectrice d'extrémité de fil (24) et présente un deuxième diamètre D ;
G) D est supérieur à d ;
H) la paroi du deuxième alésage (12) comprend, au niveau de l'extrémité avant (19) de la gaine interne (3), une arête de coupe (15) circulaire concentrique au deuxième axe d'alésage (13) ;
**caractérisé en ce que**
I) le dispositif (1) comprend un logement (5) présentant un axe central (2), une extrémité avant (26), une extrémité arrière (27) et un espace creux (8), traversant le logement (5) parallèlement à l'axe central (2), destiné à loger la gaine externe (4); et
K) l'espace creux (8) comprend, au niveau de l'extrémité avant (26) du logement (5), un troisième alésage (25) présentant un troisième axe longitudinal (29) parallèle à l'axe central (2), présentant une troisième excentricité c > 0 par rapport à celui-ci.

2. Dispositif selon la revendication 1, **caractérisé en ce que** a est supérieur à b.

3. Dispositif selon la revendication 1 ou 2, **caractérisé en ce qu'**au moins l'une des gaines (3 ; 4) est équipée, au niveau de son extrémité arrière (21 ; 22), d'un levier de manoeuvre (7) disposé transversalement aux axes d'alésage (9 ; 13).

4. Dispositif selon l'une quelconque des revendications 1 à 3, **caractérisé en ce qu'**il comprend en plus une gaine protectrice d'extrémité de fil (24) comprenant une extrémité arrière (33), une extrémité avant (34) et un alésage central (30).

5. Dispositif selon l'une quelconque des revendications 1 à 4, **caractérisé en ce qu'**un épaulement (31), présentant une surface d'appui (23) plane perpendiculaire au premier axe d'alésage (9), est formé par le rétrécissement (17), et l'extrémité avant (19) de la gaine interne (3) s'appuie contre cette surface d'appui (23).

6. Dispositif selon la revendication 4 ou 5, **caractérisé en ce que** la gaine protectrice d'extrémité de fil (24) peut être insérée parallèlement à l'axe longitudinal (11) dans le rétrécissement (17) et l'extrémité arrière (33) de la gaine protectrice d'extrémité de fil (24) peut venir s'appuyer axialement contre l'extrémité avant (19) de la gaine interne (3).

7. Dispositif selon l'une quelconque des revendications 4 à 6, **caractérisé en ce que** la paroi de l'alésage central (30) comprend, dans la gaine protectrice d'extrémité de fil (24), au niveau de l'extrémité arrière (33), une deuxième arête vive (16) concentrique à l'alésage central (30).

8. Dispositif selon l'une quelconque des revendications 1 à 7, **caractérisé en ce que** le diamètre du troisième alésage (25) correspond au diamètre extérieur de la gaine protectrice d'extrémité de fil (24), de sorte qu'une liaison serrée peut être obtenue entre la gaine protectrice d'extrémité de fil (24) et le fil métallique (20) par rotation de la gaine externe (4) dans l'espace creux (8).

9. Dispositif selon l'une quelconque des revendications 1 à 8, **caractérisé en ce que** le levier de manoeuvre (7) peut être fixé au niveau de l'extrémité arrière (21) de la gaine interne (3).

10. Dispositif selon la revendication 9, **caractérisé en ce que** l'une des gaines (3 ; 4) comprend un mécanisme d'entraînement (10) et l'autre gaine (3 ; 4) comprend un évidement (35), dans lequel le mécanisme d'entraînement (10) peut être déplacé librement à l'intérieur de l'évidement (35) lors d'une rotation des gaines (3 ; 4) l'une par rapport à l'autre selon un angle de rotation α.

11. Dispositif de coupe selon la revendication 10, **caractérisé en ce que** l'angle de rotation α se situe entre 90° et 180°.

12. Dispositif selon l'une quelconque des revendications 1 à 11, **caractérisé en ce que** le deuxième axe d'alésage (13) présente une excentricité X par rapport au deuxième axe longitudinal (11) qui est comprise entre 0,2 mm et 0,8 mm.

13. Dispositif selon la revendication 12, **caractérisé en ce que** l'excentricité X est comprise entre 0,4 mm et 0,6 mm.

14. Dispositif de coupe selon l'une quelconque des revendications 1 à 13, **caractérisé en ce que** la troisième excentricité c est comprise entre 0,05 mm et 0,5 mm.

15. Dispositif de coupe selon la revendication 14, **caractérisé en ce que** la troisième excentricité c est comprise entre 0,1 mm et 0,3 mm.
